# EUROPEAN PATENT APPLICATION

(11) **EP 4 640 747 A1**
(43) Date of publication of application: **29.10.2025**
(21) Application number: 23907174.9
(22) Date of filing: 22.12.2023
(51) Int. Cl.: C08G 77/445, C08F 299/08

(54) **RADICALLY POLYMERIZABLE POLYCAPROLACTONE-MODIFIED SILICONE COMPOUND, NOVEL SILICONE-POLYCAPROLACTONE COPOLYMER PARTICLES AND COSMETIC COMPOSITION USING SAME, AND OTHER USES**

(30) Priority: 23.12.2022 JP 2022206751
(71) Applicant: Dow Toray Co., Ltd., Tokyo 140-8617 (JP)
(72) Inventor: SUGIURA, Tsunehito, Ichihara-shi Chiba 299-0108 (JP); TANIGUCHI, Hiroko, Ichihara-shi Chiba 299-0108 (JP); TAN, Liyi, Ichihara-shi Chiba 299-0108 (JP); KANZAKI, Yasue, Ichihara-shi Chiba 299-0108 (JP)
(74) Representative: Elkington and Fife LLP
(86) International application number: PCT/JP2023/046100
(87) International publication number: WO 2024/135814

(57) **Abstract**

Problem

To provide a radical polymerizable polycaprolactone-modified silicone compound capable of forming novel silicone-polycaprolactone copolymer particles by radical polymerization using one component, and a method for producing the same. Furthermore, the present invention provides silicone-polycaprolactone copolymer particles obtained by radical polymerization of the compound, as well as a use and a production method thereof.

Solution

A radical polymerizable polycaprolactone-modified silicone compound having in each molecule two or more modified polycaprolactone structures expressed by the following structural formula (1):
(wherein n is a number ranging from 1 to 5, and Ra is a group selected from a hydrogen atom and a (meth)acrylic terminal group) and having in each molecule two or more (meth)acrylic terminal groups; silicone-polycaprolactone copolymer particles made therefrom; and use thereof.

## Description

### TECHNICAL FIELD

The present invention provides a radical polymerizable polycaprolactone-modified silicone compound having a (meth)acryl terminal group, capable of forming novel silicone-polycaprolactone copolymer particles by radical polymerization using one component, and a method for producing the same. Furthermore, the present invention relates to novel silicone-polycaprolactone copolymer particles which have a silicon atom crosslinked structure derived from the radical polymerizable polycaprolactone-modified silicone compound, as well as a polycaprolactone structure, and which can impart excellent sensation and feel during use to cosmetic materials. Furthermore, the novel silicone polycaprolactone copolymer particles have a crosslinked structure that is active with respect to biodegradability. Therefore, primary particles thereof are expected to have a property of disintegrating with the generation of non-crosslinked siloxane molecules due to a decomposition reaction of microorganisms and the like in the natural world, and thus are expected to behave as biodegradable particles. Furthermore, the present invention relates to a cosmetic raw material, cosmetic material composition, organic resin additive, and other applications containing the silicone polycaprolactone copolymer particles, as well as to a method of producing the silicone polycaprolactone copolymer particles.

### BACKGROUND ART

Silicone elastomer particles are cured from an addition reaction-curable or condensation reaction-curable silicone composition, and the particle diameter and oil absorbency thereof vary depending on the manufacturing method, but are widely used as stress-relieving agents or the like for cosmetic raw materials and thermoplastic resins. For example, as silicone particles having superior dispersibility, high lipophilicity, and superior storage stability, the present applicant has proposed silicone particles containing an alkylene group having 4 to 20 carbon atoms, which is obtained by curing a crosslinkable composition for forming silicone particles having a low amount of silicon atom-bonded hydrogen atoms per unit mass and containing an alkenyl group having 4 to 20 carbon atoms, such as a hexenyl group or the like, as described in Patent Document 1.

In addition, the applicant has focused on essential issues in conventional silicone elastomer particles. In other words, conventional silicone elastomer particles are formed through a crosslinking reaction of organopolysiloxane raw materials by hydrosilylation reactions or the like. The crosslinked structure is chemically stable, and even if these silicone elastomer particles were to be released into nature, it is undeniable that they would remain in nature without decomposing, at least for a short period of time, just like so-called microplastics. Therefore, there seems to be a latent desire in the market for silicone elastomer particles that perform well enough to smoothly replace or substitute existing silicone elastomer particles and that are expected to be highly biodegradable, in order to reduce risk to the global environment.

In view of this potential market demand, the present applicants have proposed a silicone elastomer particle having a structure crosslinked by a divalent organic group having a partial structure formed by radical polymerization of vinyl acetate as described in Patent Document 2. The silicone elastomer particles can be expected to have a high degree of biodegradability, have suppressed aggregation properties over time as compared with conventional silicone elastomer particles, and provide a smaller average secondary particle diameter. Therefore, these silicone elastomer particles have excellent dispersibility, and have excellent handling workability as a cosmetic raw material, storage stability, and blending stability in a system.

However, there is still a need for silicone elastomer particles that can impart a feel equal to or better than that of existing silicone elastomer particles and that can be expected to have high biodegradability when used as a cosmetic raw material.

On the other hand, a polycaprolactone compound is synthesized by ring-opening addition polymerization of ε-caprolactone, and is expected to have major properties as a biodegradable raw material in addition to being a film-forming material. Patent Document 3 discloses an (AB)n type block copolymer containing a polycaprolactone structure and a polysiloxane structure. However, there is no description or suggestion of silicone elastomer particles having the polycaprolactone structure, and there is no disclosure of a (meth)acrylic-modified polycaprolactone compound having a terminal group expressed by -C(=O)-CH=CH₂ or -C(=O)-CH(CH₃)=CH.

On the other hand, Non-patent Documents 1 to 3 disclose reacting a polycaprolactone compound having a polyol terminal structure with butyroyl chloride or the like, but do not disclose a radical polymerizable polycaprolactone-modified silicone compound having a specific structure, which has a plurality of polycaprolactone structures having a relatively low degree of polymerization in each molecule, where all of the terminal structures are (meth)acryl terminal groups, and can form silicone elastomer particles by a crosslinking reaction between polysiloxane structures or a radical polymerization reaction with a (meth)acryl group-containing organopolysiloxane.

### RELATED ART DOCUMENTS

### Patent Documents

Patent Document 1: International Patent Publication WO2017/191798
Patent Document 2: International Patent Publication WO2022/138346
Patent Document 3: Japanese Unexamined Patent Application 2002-146026 (Patent No. 3512399)

### Non-Patent Documents

Non-patent Document 1: Preparation of positively charged polycaprolactone film material and application of biomaterial (IWAMATSU Koji et al., Proceedings of the 2017 Nihon University Science & Engineering Department, pp. 1125 to 1126)
Non-patent Document 2: Temperature-responsive cross-linked poly ((-caprolactone) membrane that functions near body temperature (Koichiro Uto et al., Journal of Controlled Release 110 (2006) 408 to 413).
Non-patent Document 3: A novel degradable polycaprolactone network for tissue (written by HaeYong Kweon et al., Biomaterials 24 (2003) 801 to 808).

### SUMMARY OF THE INVENTION

### PROBLEM TO BE SOLVED BY THE INVENTION

An object of the present invention is to solve the aforementioned problems, and provide novel copolymer particles having a structure which is active with respect to biodegradability, and can provide texture and feel during use that is equal to or higher than conventional silicone elastomer particles when blended in a cosmetic composition or the like, a radical polymerizable polycaprolactone-modified silicone compound having a specific structure and useful as a raw material for synthesis reactions, and a method for producing the same.

Furthermore, an object of the present invention is to provide a cosmetic raw material, an organic resin additive, and other applications with excellent feel during use, and the like by using the copolymer particles. Furthermore, an object of the present invention is to provide a cosmetic material composition containing the copolymer particles, with excellent feel during use, and the like.

Furthermore, an object of the present invention is to provide: copolymer particles that, in addition to performance equal to or better than conventional silicone elastomer particles, can be expected to be biodegradable, thereby reducing potential risks to the global environment, allowing for industrially sustainable and stable use, and making it possible to promote the material as biodegradable and eco-friendly to users and consumers in general who are concerned about global environmental impact, as well as to provide a synthesis raw material, and a use thereof.

### MEANS FOR SOLVING THE PROBLEM

In order to solve the above problems, the present inventors conducted extensive research and discovered that the aforementioned problems can be resolved by a radical polymerizable polycaprolactone-modified silicone compound, comprising:
two or more modified polycaprolactone structures bonded to a silicon atom either directly or via a bivalent linking group, the structures expressed by the following structural formula (1): 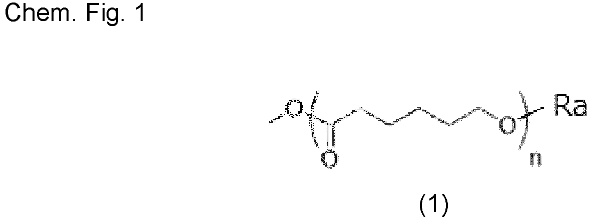
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R² is a hydrogen atom or a methyl group)};
but if the number of (meth)acrylic terminal groups contained in the modified polycaprolactone structure in the molecule is less than two, the molecule further includes two or more (meth)acrylic terminal groups expressed by -R³-CR⁴=CH₂ and bonded to silicon atoms (where R³ is a carbonyl group or a divalent linking group containing one carbonyl group, and R⁴ is a hydrogen atom or a methyl group),
and by using this compound as a raw material for silicone-polycaprolactone copolymer particles. Thereby the present invention was achieved.

Similarly, the present inventors found that the above problems can be solved by silicone-polycaprolactone copolymer particles having a structure in which at least two silicon atoms in the particles are crosslinked by a radical polymerization reaction of a radical polymerizable polycaprolactone-modified silicone compound according to any one of claim 1 to claim 4, and by cosmetic raw materials, organic resin additives, cosmetic material, and organic resins containing these particles, and thus the present invention was achieved.

### EFFECT OF THE INVENTION

The radical polymerizable polycaprolactone-modified silicone compound having a specific structure of the present invention can achieve silicone-polycaprolactone copolymer particles by radical polymerization of one component. The silicone polycaprolactone copolymer particles are blended in a cosmetic material composition or the like, and it is possible to achieve a texture and a feel during use which are equal to or higher than those of conventional silicone elastomer particles. Furthermore, the silicone polycaprolactone copolymer particles of the present invention can be used to provide a cosmetic raw material, an organic resin additive, and other applications containing the silicone elastomer particles. Furthermore, a cosmetic material composition containing the silicone polycaprolactone copolymer particles of the present invention can provide a cosmetic material with excellent feel during use, and the like.

In addition, the silicone-polycaprolactone copolymer particles of the present invention have a structure having both a polyorganosiloxane chain and polycaprolactone chain in the elastomer particles, and a divalent organic group having this partial structure is active in a biodegradable reaction, and is designed so that in a biodegradable environment, the crosslinked structure formed between silicon atoms in the copolymer particles is at least partially cleaved, and the primary particles are disintegrated in conjunction with the generation of polyorganosiloxane having a non-crosslinked structure. Therefore, the elastomer particles of the present invention are expected to be biodegradable, which reduces the risk to the global environment, and appeal to users and consumers in general, who are concerned about global environment impact, as an eco-friendly material that can be used with a considerable sense of ease.

### Preferred Mode for Carrying Out the Invention

In the present specification, the term "(meth)acryl" refers to "acrylic or methacrylic", and when expressed as "(meth)acryl-modified" indicates that the modifying group may be one or both of an acryl modifying group and a methacryl modifying group. Similarly, the term "(meth) acryloxy" refers to "methacryloxy or acryloxy" and "(meth)acryloxy group-containing organic group" indicates either one or both of methacryloxy group-containing organic groups and acryloxy group-containing organic groups.

Radical polymerizable polycaprolactone-modified silicone compound The radical polymerizable polycaprolactone-modified silicone compound of the present invention is designed as a reactive raw material for the novel silicone-polycaprolactone copolymer particles of the present invention, and is a component that imparts a crosslinking structure by a radical polymerization reaction using divalent organic groups between polycaprolactone chains and silicone chains in the elastomer particles.

Specifically, the radical polymerizable polycaprolactone-modified silicone compound of the present invention has two or more modified polycaprolactone structures bonded to a silicon atom either directly or via a divalent linking group, the structures expressed by the following structural formula (1): 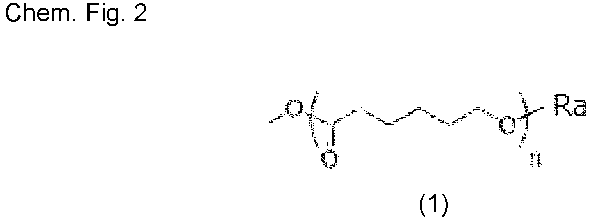
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R² is a hydrogen atom or a methyl group)}; and if the number of (meth)acrylic terminal groups contained in the modified polycaprolactone structure in the molecule is less than two, the molecule further includes two or more (meth)acrylic terminal groups expressed by -R³-CR⁴=CH₂ and bonded to silicon atoms (where R³ is a carbonyl group or a divalent linking group containing one carbonyl group, and R⁴ is a hydrogen atom or a methyl group).

In other words, the present radical polymerizable polycaprolactone-modified silicone compound has two or more (meth)acrylic terminal groups as a portion of the modified polycaprolactone structure in a molecule or as functional groups different from the modified polycaprolactone structure, and the component alone has radical polymerizability.

Specifically, the above Ra may be a hydrogen atom. In this case, the molecule has a modified polycaprolactone structure having an OH terminal, but in order to impart radical polymerizability to the molecule as a whole, the molecule must have a prescribed functional group having a (meth)acrylic terminal group bonded to a silicon atom, which is different from the modified polycaprolactone structure. On the other hand, when the above Ra is a prescribed functional group having a (meth)acrylic terminal group, the modified polycaprolactone structure itself imparts radical polymerizability, so this component alone can provide radical polymerizability for the whole molecule, even without having any other radical polymerizable functional groups.

Furthermore, from the viewpoint of achieving biodegradability of the novel silicone-polycaprolactone copolymer particles obtained by radical polymerization of the present radical polymerizable polycaprolactone-modified silicone compound, specifically, the property of disintegration in conjunction with the generation of a non-crosslinked polyorganosiloxane having a chain polysiloxane structure upon cleavage of the reaction site, the polysiloxane structure in the molecule is preferably chain-like, more preferably linear or branched, and particularly preferably has a linear polysiloxane structure.

The modified polycaprolactone structure bonded to a silicon atom and expressed by structural formula (1), or the (meth)acrylic terminal group bonded to a silicon atom and expressed by -R³-CR⁴=CH₂ (where R³ is a carbonyl group or a divalent linking group containing one carbonyl group, and R⁴ is a hydrogen atom or a methyl group) may be bonded to the terminus or side chain of the linear polysiloxane structure.

For example, radical polymerizable polycaprolactone-modified silicone compounds having the structure shown below are included in the scope of the present invention. In the formula, x is a number ranging from 1 to 5, and n and m are both positive numbers of 2 or more. The functional groups bonded to the line (-) on the silicon atom are not particularly limited so long as being a monovalent organic group that does not impair the technical effects of the present invention. In industrial use, the functional group may be a methyl group, a phenyl group, a hydroxyl group, or the like, or may be a monovalent organic group containing a polyoxyalkylene group, a macromonomer structure having a silicon atom, or a carbosiloxane dendrimer structure.

Herein, of these structural examples, the top four are examples of molecular structures having a modified polycaprolactone structure bonded to a silicon atom at the end of a linear polysiloxane structure, and the bottom three are examples of molecular structures having a modified polycaprolactone structure bonded to a silicon atom in a side chain of a linear polysiloxane structure. In all of the structural examples, the radical polymerizable polycaprolactone-modified silicone compound has two or more (meth)acrylic terminal groups in each molecule.

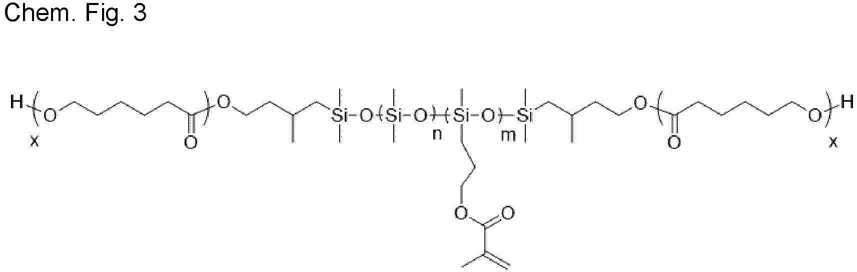

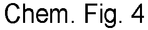

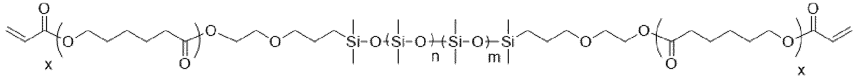

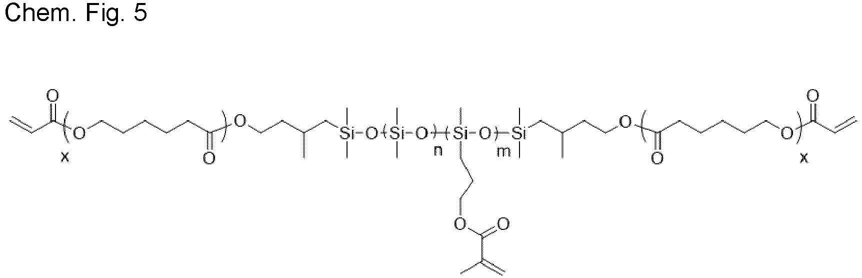

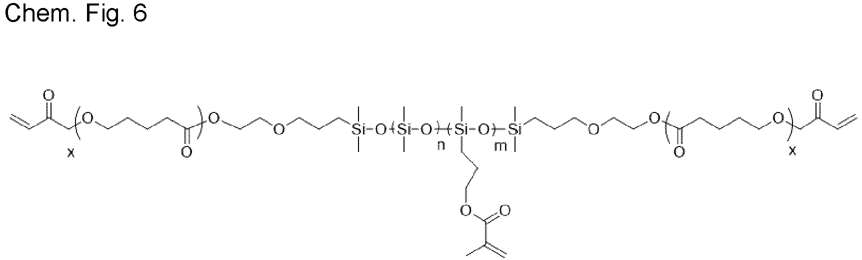

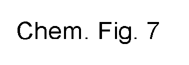

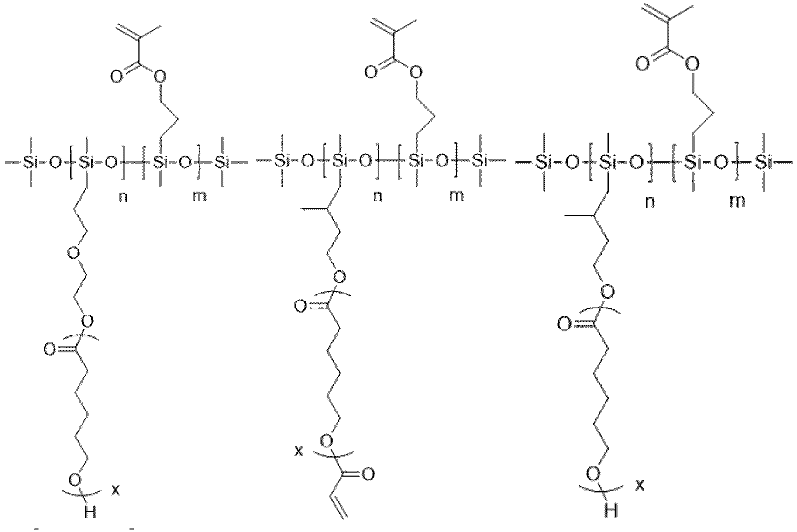

More specifically, the radical polymerizable polycaprolactone-modified silicone compound of the present invention has a straight chain or branched chain polysiloxane structure, wherein the molecular chain terminal or molecular chain side chain includes:
two or more modified polycaprolactone structures bonded to a silicon atom either directly or via a bivalent linking group, the structures expressed by the following structural formula (1): 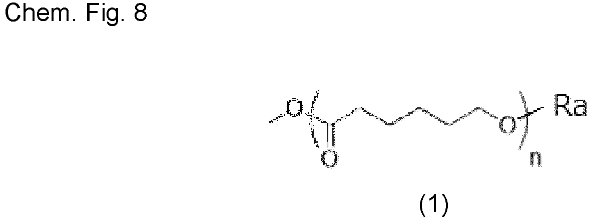
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R² is a hydrogen atom or a methyl group)};
and if the number of (meth)acrylic terminal groups contained in the modified polycaprolactone structure in the molecule is less than two, the molecule includes two or more (meth)acrylic terminal groups expressed by -R³-CR⁴=CH₂ and bonded to silicon atoms (where R³ is a carbonyl group or a divalent linking group containing one carbonyl group, and R⁴ is a hydrogen atom or a methyl group).

Particularly preferably, the radical polymerizable polycaprolactone-modified silicone compound of the present invention has a linear polysiloxane structure having modified polycaprolactone structures at both terminals of the molecular chain. In other words, the radical polymerizable polycaprolactone-modified silicone compound is preferably expressed by the following structural formula (2): 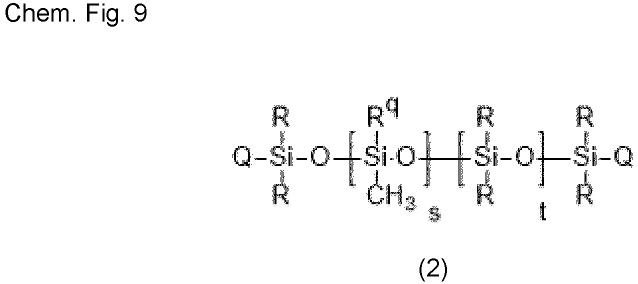
{where Q is a modified polycaprolactone structure expressed by the following structural formula (1), bonded to a silicon atom either directly or through a divalent linking group: 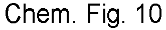 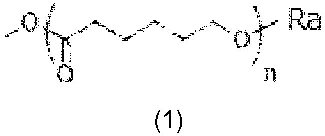
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R₂ is a hydrogen atom or a methyl group)};
R^{q} is a (meth)acrylic terminal group bonded to a silicon atom and expressed by -R³-CR⁴=CH₂ (where R³ is a carbonyl group or a divalent linking group containing one carbonyl group, and R⁴ is a hydrogen atom or a methyl group); R is an alkyl group or an aryl group having 1 to 20 carbon atoms;
s is a number ranging from 0 to 50, and t is a number ranging from 1 to 500; however, when Ra is a hydrogen atom in one or both of the terminal groups Q, s is a positive number and indicates having at least two (meth)acrylic terminal groups in the molecule}.

Here, from the viewpoint of having at least two modified polycaprolactone structures and a (meth)acrylic terminal group (which may be a part of the modified polycaprolactone structure) in the molecule, the radical polymerizable polycaprolactone-modified silicone compound expressed by the above structural formula (2) is preferably a radical polymerizable polycaprolactone-modified silicone compound that satisfies either of the following conditions (I) or (II), depending on the presence or absence of a (meth)acrylic terminal group in the modified polycaprolactone structure.
(I) In both terminal groups Q, Ra is a hydrogen atom, s is a number in a range of 2 to 50, and t is a number in a range of 1 to 500;
(II) In both of the terminal groups Q, Ra is a (meth)acrylic terminal group expressed by -R¹-CR²=CH₂ (where R¹ is a carbonyl group or a divalent linking group containing one carbonyl group, and R² is a hydrogen atom or a methyl group), s is a number in a range of 0 to 50, and t is a number in a range of 1 to 500.

In the radical polymerizable polycaprolactone-modified silicone compound of the present invention, the modified polycaprolactone structure (Q) expressed by structural formula (1) is bonded to a silicon atom directly or via a divalent linking group. Here, the divalent linking group is preferably a linear or branched alkylene group having from 2 to 20 carbon atoms.

In structural formula (1), n in the formula represents the number of repeating caprolactone units {-C(=O)-C₅H₁₀-O-} in the structure, and is a number in a range of 1 to 5, and may be a number in a range of 1 to 3. The radical polymerizable polycaprolactone-modified silicone compound of the present invention is designed as a crosslinking agent between silicon atoms in the particles, and has the advantage that the sensation and feel of use derived from the organopolysiloxane main chain of the copolymer particles is not significantly impaired because the number of caprolactone units in each structure is relatively small and the number of repeating caprolactone units in the molecule as a whole is also relatively small.

In the radical polymerizable polycaprolactone-modified silicone compound of the present invention, in addition to the fact that the number of repeating caprolactone units in each structure is relatively small, the sum of the numbers of repeating caprolactone units in the molecule is preferably in a range of 2 to 20, and may be in a range of 2.5 to 15, or in a range of 3.0 to 10. If the number of repeating caprolactone units in the radical polymerizable polycaprolactone-modified silicone compound molecule exceeds the upper limit, the properties derived from the polycaprolactone structure are strongly reflected in the obtained silicone elastomer particles, which may adversely affect the texture and feel during use of the cosmetic material or the like.

In structural formula (1), Ra is a hydrogen atom or a (meth)acrylic terminal group expressed by -C(=O)-R¹-CR²=CH₂.

Ra being a hydrogen atom indicates a structure derived from a polycaprolactone compound having a polyol (alcoholic) hydroxyl terminal group, and thus the modified polycaprolactone structure itself does not have radical polymerizability. On the other hand, when Ra is a (meth)acrylic terminal group of the above structure, the modified polycaprolactone structure itself has radical polymerizability. Here, R¹ is a carbonyl group or a divalent linking group containing one carbonyl group, and is preferably a group selected from carbonyl groups (-C(=O)-), a divalent organic group having 1 to 20 carbon atoms containing a carbonyl group, or a divalent silicon atom-containing group containing a carbonyl group. Furthermore, R² is a hydrogen atom or a methyl group, and provides an acryl-modified group or a methacryl-modified group.

The radical polymerizable polycaprolactone-modified silicone compound of the present invention is a single component, and forms a crosslinked structure in the copolymer particles by a radical polymerization reaction, and when the obtained silicone-polycaprolactone copolymer particles are used as a cosmetic raw material, there is expectation that the feel of use or the touch sensation will not be impaired, and that the crosslinked structure formed between two silicon atoms will be biodegradable.

The radical polymerizable polycaprolactone-modified silicone compound of the present invention has two or more reactive (meth)acrylic modifying groups, and therefore can form a crosslinked structure between silicon atoms in the molecules, as a result of the radical polymerization reaction. As described above, the (meth)acrylic modifying group may be present in the modified polycaprolactone structure, or may be a (meth)acrylic modifying group bonded to a side chain.

The method for producing (method for synthesizing) the radical polymerizable polycaprolactone-modified silicone compound of the present invention is not particularly limited, but a modified polycaprolactone structure is preferably introduced into an organohydrogenpolysiloxane by a hydrosilylation reaction and, if necessary, a (meth)acrylic modifying group is introduced into the polyol-based (alcohol-based) hydroxyl group terminal in the modified polycaprolactone structure.

Specifically, the radical polymerizable polycaprolactone-modified silicone compound of the present invention can be synthesized by a method for producing the radical polymerizable polycaprolactone-modified silicone compound, including a step of:
performing a hydrosilylation reaction between
(a) an organohydrogenpolysiloxane having at least 2 silicon-bonded hydrogen atoms in each molecule; and
(b) a polycaprolactone compound having a vinyl terminal structure expressed by the following structural formula (1') 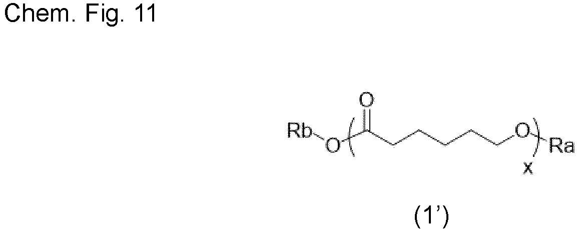
   {wherein x is a number ranging from 1 to 5,
   Ra is a group selected from hydrogen atoms and (meth)acrylic terminal groups expressed by -R₁-CR²=CH₂ (where R¹ is a carbonyl group or a divalent linking group containing one carbonyl group, and R² is a hydrogen atom or a methyl group), and
   Rb is CH₂=CH(Rc)-R'- (Rc is a hydrogen atom or a methyl group, and R' is a divalent organic group having 1 to 20 carbon atoms which may contain an oxygen atom)}
   in the presence of
(c) a hydrosilylation reaction catalyst.

In structural formula (1'), Rb forms a divalent linking group that links the modified polycaprolactone structure or a precursor structure thereof according to structural formula (1) described above to the polysiloxane structure by a hydrosilylation reaction between a silicon-bonded hydrogen atom of the organohydrogenpolysiloxane which is component (a), and a terminal vinyl group of Rb. For example, in the finally obtained radical polymerizable polycaprolactone-modified silicone compound, if the modified polycaprolactone structure of structural formula (1) or a precursor structure thereof and the polysiloxane structure have a structure bonded via a linear or branched alkylene group, R' may be an alkylene group having 1 to 20 carbon atoms, and is particularly preferably an alkylene group having 1 to 10 carbon atoms.

In the structural formula (1'), if Ra is a hydrogen atom, a modified polycaprolactone structure having a polyol (alcohol) hydroxyl group terminal is introduced into the molecule by the above reaction. Meanwhile, as described above, the finally obtained radical polymerizable polycaprolactone-modified silicone compound must have two or more (meth)acrylic terminal groups. Therefore, if a (meth)acrylic terminal group is not separately introduced into the raw material which is component (a), a (meth)acrylic terminal group must be introduced into the hydroxyl group terminus.

In this case, a (meth)acrylic terminal group expressed by -R¹-CR²=CH₂ (where R¹ is a carbonyl group or a divalent linking group containing one carbonyl group, and R² is a hydrogen atom or a methyl group) can be introduced into the modified polycaprolactone structure by further performing a step (d) of reacting the modified polycaprolactone structure having a hydroxyl group terminal with a (meth)acryloyl chloride compound expressed by Cl-C(=O)-R¹-CR²=CH₂ (R¹ is a chemical bond between CH and C(=O) or a divalent organic group having 1 to 20 carbon atoms, and R² is a hydrogen atom or a methyl group) in the presence of a basic catalyst.

Note that the basic catalyst that can be used in the reaction is not particularly limited, and may be an alkali metal salt of an inorganic base such as sodium hydroxide, potassium hydroxide, calcium hydroxide, sodium carbonate, potassium carbonate or sodium hydrogen carbonate; an amine compound such as triethylamine, pyridine or dimethylaminopyridine; or a nitrogen-containing heterocyclic compound.

The organohydrogenpolysiloxane having at least two silicon-bonded hydrogen atoms in the molecule, which is component (a), is a component that determines the polysiloxane structure of the radical polymerizable polycaprolactone-modified silicone compound of the present invention, but from the viewpoint of achieving biodegradability of the novel silicone-polycaprolactone copolymer particles obtained by radical polymerization of the present radical polymerizable polycaprolactone-modified silicone compound, specifically, the property of disintegration in conjunction with the generation of a non-crosslinked polyorganosiloxane having a chain polysiloxane structure upon cleavage of the reaction site, the polysiloxane structure in the molecule is an organohydrogenpolysiloxane that preferably has a chain-like structure, more preferably a linear or branched structure, and particularly preferably has a linear polysiloxane structure. Furthermore, in the present invention, the organohydrogenpolysiloxane is particularly preferably a linear organohydrogenpolysiloxane having silicon-bonded hydrogen atoms at both molecular chain terminals.

Furthermore, in the organohydrogenpolysiloxane of component (a), the (meth)acrylic terminal group is preferably introduced in advance into the side chain moiety by, for example, an equilibrium reaction between a (meth)acrylic-modified silane (for example, (meth)acryloxypropylmethyldialkoxysilane) and the organohydrogenpolysiloxane, in the presence of an acid catalyst, and the like. Specifically, the aforementioned component (a) is preferably (a') an organohydrogenpolysiloxane having in the molecule at least two silicon-bonded hydrogen atoms and a (meth)acrylic terminal group bonded to a silicon atom and expressed by -R³-CR⁴=CH₂ (where R³ is a carbonyl group or a divalent linking group containing one carbonyl group, and R⁴ is a hydrogen atom or a methyl group). By introducing a modified polycaprolactone structure into the organohydrogenpolysiloxane having a (meth)acrylic terminal group in the step described above, a radical polymerizable polycaprolactone-modified silicone compound having a (meth)acrylic terminal group in a side chain moiety, or a precursor thereof, can be obtained, independent of the modified polycarbonate structure. In the reaction, usable acidic catalysts include, but are not limited to, trifluoromethanesulfonic acid, sulfuric acid, hydrochloric acid and the like.

The hydrosilylation catalyst used in the above method of production is not particularly limited, but is preferably a hydrosilylation reaction catalyst containing platinum metal, such as platinic acid chloride, alcohol-modified platinic acid chloride, olefin complexes of platinic acid chloride, complexes of platinic acid chloride with ketones, complexes of platinic acid chloride with vinyl siloxane, platinum tetrachloride, platinum fine powder, platinum supported on an alumina or silica carrier, platinum black, olefin complexes of platinum, alkenylsiloxane complexes of platinum, carbonyl complexes of platinum, and thermoplastic organic resin powders such as methyl methacrylate resin, polycarbonate resin, polystyrene resin, and silicone resin containing these platinum-based catalysts. In particular, platinum alkenylsiloxane complexes such as a complex of platinum chloride with divinyltetramethyldisiloxane, a complex of platinum chloride with tetramethyltetravinylcyclotetrasiloxane, a platinum divinyltetramethyldisiloxane complex, and a platinum tetramethyltetravinylcyclotetrasiloxane complex can be preferably used. Note, as the catalyst for promoting the hydrosilylation reaction, a non-platinum based metal catalyst such as iron, ruthenium, iron/cobalt, or the like may be used. The amount used may be any catalytic amount, and typically, the amount is preferably such that the amount of platinum-based metal contained in component (c) is in a range of 1 to 1,000 ppm, and more preferably in a range of 5 to 500 ppm, relative to the total mass of the above radical polymerizable polycaprolactone-modified silicone compound.

In the synthesis reaction of the radical polymerizable polycaprolactone-modified silicone compound, a chain transfer agent can be added optionally. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

The polycaprolactone compound having a vinyl group terminal structure expressed by structural formula (1'), which is component (b), can be obtained by ring-opening addition polymerization of a vinyl group-containing alcohol and ε-caprolactone, and thus a polycaprolactone compound having a terminal hydroxyl group (for example, a compound in which Ra is a hydrogen atom) can be obtained. Note that a polycaprolactone compound having a vinyl group terminal structure with a (meth)acrylic terminal group as Ra can be obtained by reacting the terminal hydroxyl group with a (meth)acryloyl chloride compound in the presence of a basic catalyst. In this case, a radical polymerizable polycaprolactone-modified silicone compound having a modified polycaprolactone structure with a (meth)acrylic terminal group can be obtained in a one-step reaction by the hydrosilylation reaction of the aforementioned component (a) and component (b).

The basic catalyst that can be used in this reaction is the same as described above.

The reaction for introducing the (meth)acrylic terminal group to the polycaprolactone can be performed in an organic solvent, and examples of organic solvents that can be used in the reaction include ketones such as acetone, methyl ethyl ketone, methyl isobutyl ketone and cyclohexanone; amides such as formamide, acetamide, N-methyl-2-pyrrolidone (NMP), N,N-dimethylformamide, and dimethylacetamide; halogenated hydrocarbons such as methylene chloride, chloroform, 1,2-dichloroethane, chlorobenzene, bromobenzene, dichlorobenzene, benzotrifluoride and hexafluoro-2-propanol; sulfoxides such as dimethyl sulfoxide (DMSO), diethyl sulfoxide and benzylphenyl sulfoxide; ethers such as diethyl ether, diisopropyl ether, dibutyl ether, tetrahydrofuran (THF), dioxane, 1,2-dimethoxyethane and cyclopentylmethyl ether; esters such as ethyl acetate; nitriles such as acetonitrile and benzonitrile; aromatic hydrocarbons such as benzene, toluene and xylene; and mixtures of two or more of these.

In the reaction, one or more polymerization inhibitors may be contained in the system in order to prevent the synthesized (meth)acryl-modified polycaprolactone compound from further undergoing a radical polymerization reaction. Examples include one or more types selected from hindered phenol-based polymerization inhibitors, hydroquinone-based polymerization inhibitors, and catechol-based polymerization inhibitors.

The reaction conditions should be appropriately selected based on the synthesis amount, the reaction apparatus, and the like, but the (meth)acryloyl chloride compound is added dropwise while stirring a mixed solution containing the polycaprolactone compound, the basic catalyst, and the optional polymerization inhibitor under a flow of an inert gas such as nitrogen. Note that after completion of the reaction, it is particularly preferable to mutually separate the intended (meth)acryllic-modified polycaprolactone compound, and to distill off the unnecessary organic solvent under reduced pressure for purification.

### Novel silicone-polycaprolactone copolymer particles

The radical polymerizable polycaprolactone-modified silicone compound of the present invention was developed as a raw material for producing, through a radical polymerization reaction, novel silicone-polycaprolactone copolymer particles (hereinafter sometimes simply referred to as "copolymer particles") that have the same or better feel and performance as conventional silicone elastomer particles, and that are also biodegradable. The copolymer particles, applications thereof including cosmetic raw materials in particular, a production method thereof, as well as a cosmetic material composition and an organic resin (including paint and coating agent) containing the same will be described in detail below.

The silicone-polycaprolactone copolymer particles of the present invention have a structure in which at least two silicon atoms in the particle are crosslinked by the radical polymerization reaction of the above-mentioned radical polymerizable polycaprolactone-modified silicone compound.

The copolymer particles of the present invention preferably further contain a polyorganosiloxane structure expressed by:

-(R¹₂SiO)ₙ-

(where R¹ represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and n is a number ranging from 1 to 1000).
The structure is a linear polysiloxane structure derived from the radical polymerizable polycaprolactone-modified silicone compound described above, and imparts appropriate hardness and flexibility to the resulting copolymer particles.

The copolymer particles of the present invention are preferably obtained by curing crosslinking reactive emulsified particles by a crosslinking reaction. Particularly preferably, the copolymer particles of the present invention are defined by the production process, and may be silicone-polycaprolactone copolymer particles obtained by radical polymerization of 100 parts by mass of (A) the above-mentioned radical polymerizable polycaprolactone-modified silicone compound, in the presence of 0.1 to 10 parts by mass of (B) a radical polymerization initiator.

Particularly preferably, the copolymer particles of the present invention may be silicone-polycaprolactone copolymer particles obtained by crosslinking in water crosslinkable emulsion particles containing at least (A) 100 parts by mass of the above-mentioned radical polymerizable polycaprolactone-modified silicone compound, and (B) 0.1 to 10 parts by mass of a radical polymerization initiator, where the crosslinkable emulsion particles are obtained by emulsifying in water a crosslinkable silicone composition that can be crosslinked by a radical polymerization reaction.

Component (A) is as described above. Component (B) is a radical polymerization initiator, which is a component that promotes the radical polymerization reaction of the radical polymerizable polycaprolactone-modified silicone compound, and is a conventionally known compound. Specific examples of component (B) include azo compounds such as 2,2'-azobis(isobutyronitrile), 2,2'-azobis(2-methylbutyronitrile), and 2,2'-azobis(2,4-dimethylvaleronitrile); organic peroxides such as benzoyl peroxide, lauroyl peroxide, tert-butyl peroxybenzoate, tert-butylperoxy-2-ethylhexanoate, and tert-hexylperoxy-2-ethylhexanoate; and persulfates such as potassium persulfate, sodium persulfate, ammonium persulfate, and the like. One type of these radical initiators may be used alone, or two or more types may be mixed and used together. For reasons described below, a water-soluble persulfate such as potassium persulfate is conveniently used as component (B).

The amount of the radical polymerization initiator used is in a range from 0.1 to 10 parts by mass, and preferably from 0.1 to 5 parts by mass, per 100 parts by mass of the total of component (A). In particular, if component (B) is a water-soluble persulfate such as potassium persulfate or the like, the addition and reaction of component (B) is extremely easy when subjecting crosslinkable emulsion particles obtained by emulsifying the above-mentioned radical polymerizable polycaprolactone-modified silicone compound in water to a radical polymerization reaction in water. Furthermore, upon completion of the radical polymerization reaction, it is particularly desirable to add aminomethylpropanediol or the like in a range of 0.1 to 5 parts by mass, for the purpose of stopping the reaction and neutralizing the solution by pH adjustment.

Furthermore, copolymer particles obtained via such a production process may further improve the appearance, spreadability, and tactile sensation of the cosmetic material, particularly when used as a cosmetic raw material, and particles obtained via this production method tend to be more suitable for solving the problem of the present invention. Thus, one suitable form for achieving the technical effect of the present invention can be and is suitably defined by the manufacturing process.

The copolymer particles of the present invention are not particularly limited in terms of the average primary particle diameter thereof, but from the perspective of imparting a smooth texture and feel during use to the cosmetic material, not causing appearance defects and the like, storage stability and blending stability as a cosmetic raw material, and the like, the average primary particle diameter measured by a laser diffraction scattering method is preferably in a range of 0.5 to 20 µm, more preferably in a range of 0.5 to 15 µm. Note that the particle size of the silicone-polycaprolactone copolymer particles can be controlled by the steps of crushing/classifying the emulsified particles of the radical polymerizable polycaprolactone-modified silicone compound and the resulting copolymer particles.

The shape of the copolymer particles of the present invention includes, for example, spherical, regular spherical shape, elliptical, and irregular shapes, and in particular, spherical and regular spherical shapes are preferred. Spherical elastomer particles can be easily obtained by the method described later, in which the particles are prepared in the form of an aqueous suspension and dried using a vacuum dryer, hot air circulation oven, or spray dryer.

Furthermore, in the present invention, the radical polymerizable polycaprolactone-modified silicone compound used to form the silicone polycaprolactone copolymer particles is preferably in a range of 10 to 80 when cured in a sheet form, as measured by a JIS A hardness tester as defined in JIS K6301. If the JIS-A hardness of the rubber sheet measured by curing the crosslinking reactive silicone composition into a sheet form is in a range above, the resulting elastomer particles will have sufficiently low aggregation and will tend to have exceptional flowability, dispersibility, smoothness, silkiness, and a soft texture. Furthermore, by selecting the JIS-A hardness described above, it is possible to design or predict to some extent the feel during use, texture, and handling workability when added to a cosmetic material, and to improve stress relaxation properties when added to an organic resin. Note that when the copolymer particles of the present invention are used as a cosmetic raw material or stress relief agent or the like for organic resins, copolymer particles having a JIS-A hardness in a range of 30 to 80, and particularly 50 to 80, described above are particularly preferably used.

Optionally, the copolymer particles of the present invention may further contain a structure in which some or all of the surface thereof is covered with one or more types selected from organopolysiloxane resins, silica, and other silicone elastomer particles. Such coating may be expected to further reduce aggregation, control oil absorbency, improve texture, and the like.

Optionally, the copolymer particles of the present invention may be mesoporous structures with micropores.

Optionally, the copolymer particles of the present invention may contain an oil agent that is liquid at 40°C. The oil agent can be easily included in the copolymer particles by emulsifying together in the crosslinking reactive composition described later, and the inclusion of the oil agent may be expected to further reduce aggregation, control oil absorbency, improve texture, and the like. In consideration of biodegradability, a biodegradable oil such as olive oil or the like may be added.

Optionally, when biodegradability is the main objective of the copolymer particles of the present invention, the copolymer particles of the present invention are preferably substantially free of a structure containing a silalkylene group.

The silicone-polycaprolactone copolymer particles of the present invention are copolymers obtained using the above-mentioned radical polymerizable polycaprolactone-modified silicone compound as a radical polymerizable monomer component that itself forms a copolymer structure by radical polymerization, and do not significantly impair the sensation and feel of use, are active in biodegradable reactions, and have a property that the crosslinked structures formed between silicon atoms in the copolymer particles are at least partially cleaved in a biodegradable environment, and the primary particles disintegrate in conjunction with the generation of polyorganosiloxanes with a non-crosslinked structure, as compared to conventional silicone elastomer particles. In particular, if the radical polymerizable polycaprolactone-modified silicone compound has a linear polyorganosiloxane structure, the biodegradable reaction facilitates the breakdown of the copolymer particles into linear polyorganosiloxane molecules, which are broken down into fine liquid components, not solid powders with minute particle diameter sizes, as in microplastics. Therefore, it is expected to have little impact or burden on the global environment, as it is unlikely to cause problems of bioaccumulation through the food chain or accumulation/deposition in the environment.

### Forming silicone-polycaprolactone copolymer particles and method of production

The copolymer particles of the present invention can be produced by a method including a step of curing crosslinkable emulsion particles obtained by emulsifying in water a crosslinkable composition containing the aforementioned radical polymerizable polycaprolactone-modified silicone compound in the presence of a radical polymerization initiator to obtain spherical copolymer particles.

More specifically, the copolymer particles of the present invention can and preferably are prepared using a production method including the following steps (I) and (II).
Step (I): a step of emulsifying (A) the radical polymerizable polycaprolactone-modified silicone compound in water, in the presence of (B) a radical polymerization initiator, to form crosslinkable reactive emulsion particles;
Step (II): a step of curing the crosslinkable emulsified particles obtained in step (I) in the presence of a radical initiator to obtain silicone-polycaprolactone copolymer particles.

The crosslinkable composition containing the radical polymerizable polycaprolactone-modified silicone compound used to form the copolymer particles can be mixed uniformly using the mechanical force of a mixer or the like. Note that the crosslinkable composition may contain an oil agent that is liquid at 40°C. The oil agent can be finally incorporated into the copolymer particles by emulsifying in the crosslinkable composition. Furthermore, in consideration of biodegradability, dilution with a biodegradable oil such as olive oil is possible.

In this method, silicone elastomer particles can be obtained by emulsifying and curing the aforementioned crosslinkable composition in a surfactant aqueous solution. In addition, the particle diameter can be easily adjusted by adjusting the emulsion particle diameter. Examples of these surfactants include nonionic, anionic, cationic, betaine types, as well as water soluble polymers such as polyvinylalcohols and the like. The particle diameter of the obtained copolymer particles will differ according to the type and content of the surfactant. In order to prepare copolymer particles having a small particle diameter, the amount of this surfactant added should be in a range of 0.5 to 50 mass parts per 100 mass parts of the crosslinkable composition.

An emulsifier is preferably used to uniformly disperse the crosslinking composition in water in the form of crosslinking reactive emulsified particles. Examples of the emulsifying machine include a homomixer, a paddle mixer, a Henschel mixer, a homo-disper, a colloid mill, a propeller agitator, a homogenizer, an in-line continuous emulsifier, an ultrasonic emulsifier, and a vacuum kneading machine.

The aqueous dispersion of the crosslinking reactive emulsified particles prepared by the method above can then be heated or left at room temperature to cure the crosslinking reactive emulsified particles in the aqueous dispersion to prepare an aqueous dispersion of copolymer particles. When such an aqueous dispersion is heated, the heating temperature is preferably 100°C or lower from the perspective of hydrosilylation reactivity or radical polymerization reactivity, and is particularly preferably 10 to 95°C. Furthermore, examples of a method of heating the aqueous dispersion containing the crosslinking reactive emulsified particles include a method of directly heating the water-based dispersion, and a method of adding the aqueous dispersion to hot water. The crosslinking reaction causes the liquid crosslinking reactive emulsified particles to cure in water, forming an aqueous dispersion of copolymer particles.

The resulting silicone-polycaprolactone copolymer particles of the present invention can be used as-is as an aqueous dispersion (aqueous suspension). In particular, the aqueous suspension form may be and is preferably used in cosmetic raw materials and the like. When added to a cosmetic material that uses an aqueous solution as a dispersion medium (such as hair cosmetic materials and the like), the copolymer particles may be easily and uniformly dispersed to achieve a desired performance and feel during use by being added as an aqueous dispersion containing the copolymer particles of the present invention.

Preferably, the silicone polycaprolactone copolymer particles of the present invention can be isolated by removing water from the aqueous dispersion of copolymer particles. The method of removing water from the aqueous dispersion includes, for example, drying using a vacuum dryer, a hot air circulation oven, or a spray dryer. Note that the heating and drying temperature of the spray dryer must be set appropriately based on the heat resistance, crosslinking temperature, and the like of the silicone elastomer particles. Note that in order to prevent secondary aggregation of the resulting microparticles, the temperature of the copolymer particles is preferably controlled to be equal to or lower than the glass transition temperature thereof. The copolymer particles thus obtained can be recovered by a cyclone, a bag filter, or the like. Note that as a pretreatment for this operation, the dispersion may be concentrated by a method of heating and dehydration, filtration separation, centrifugation, decantation, or the like, and if necessary, the dispersion may be washed with water.

The silicone polycaprolactone copolymer particles of the present invention may be surface treated, if necessary, to further improve the aggregation suppression effect of the copolymer particles of the present invention. Furthermore, surface treatment with another known hydrophilic or hydrophobic treatment agent or the like may be applied. Optionally, the resulting copolymer particles may be further coated with silica or other inorganic microparticles, silicone resin, or the like, in whole or in part on the surface thereof, as described above. Furthermore, the resulting copolymer particles may be crushed or pulverized by a mechanical force if necessary, or classified using a known technique.

### Cosmetic Raw Material and Cosmetic Material Composition

The silicone polycaprolactone copolymer particles of the present invention are useful as cosmetic raw materials, and when blended in a cosmetic composition or the like, the particles are flexible and improve the feel and texture of the cosmetic materials and the like, and provide outstanding handling workability, storage stability, and blending stability in systems as a cosmetic raw material.

In particular, the silicone polycaprolactone copolymer particles of the present invention are superior to known silicone particles in terms of feel during use and texture, have a higher degree of freedom in formulation design, do not absorb oily raw materials over time when formulated in cosmetic materials, do not cause thickening or changes in texture, and when applied to the skin or hair, do not make the cosmetic material feel greasy or sticky. In addition, when the silicone polycaprolactone copolymer particles of the present invention are used in combination with a UV protection component, the UV protection effect of the cosmetic material may be improved without impairing the texture and feel during use of the cosmetic material as compared with other powders or existing silicone elastomer particles.

Furthermore, the silicone polycaprolactone copolymer particles of the present invention are active with respect to biodegradable reactions while providing performance that is equal or superior to conventionally known silicone elastomer particles. Moreover, a crosslinked structure formed between silicon atoms in the particle is at least partially cleaved in a biodegradable environment, and primary particles have a property of disintegrating with the generation of a polyorganosiloxane with a non-crosslinked structure. Therefore, the silicone elastomer particles are a low risk and low impact material for the global environment. Furthermore, use is possible as a replacement to conventionally known silicone elastomer particles, making it extremely versatile.

The cosmetic material composition containing the silicone polycaprolactone copolymer particles of the present invention are not particularly limited in type, and examples include products such as soaps, body shampoos, facial cleansing creams, and other cleaning cosmetic materials; toners, creams/milky lotions, packs, and other base cosmetic products; powders, foundation, and other base makeup cosmetic materials; lipstick, cheek rouge, eye shadow, eyeliners, mascara, and other facial cosmetic materials; nail polish and other makeup cosmetic materials; shampoos, hair rinses, hairdressing material, hair growth promoters, hair dye, and other hair cosmetic materials; perfume and eau de cologne, and other aromatic cosmetic materials; toothpastes; bath agents; and hair removal agents, shaving lotions, antiperspirants/deodorants, sunscreen agents, and other special cosmetic materials. Examples of the dosage forms of these cosmetic material compositions include aqueous liquid, oil-based liquid, emulsion, cream, foam, semi-solid, solid, and powder. These cosmetic material compositions can also be used by spraying.

In these cosmetic material compositions, the amount of the silicone polycaprolactone copolymer particles described above is preferably within a range of 0.5 to 99.0 mass% in the cosmetic material composition, and particularly preferably within a range of 1.0 to 95 mass%. This is because if the amount of the copolymer particles described above exceeds the upper limit of a range above, the effect as a cosmetic material is lost, and if the amount is below the lower limit of a range above, feel during use and the like of the cosmetic material composition and the like is less likely to be improved.

With respect to cosmetic material compositions (in particular, each formulation example) containing silicone particles (silicone rubber powder and the like) or silicone composite particles, which have been proposed in Japanese Unexamined Patent Application H07-316014, International Patent Publication WO2017/191798, International Patent Application PCT/JP2021/46142, Japanese Unexamined Patent Application H02-243612, Japanese Unexamined Patent Application 2002-146026 (Patent 3512399), Japanese Unexamined Patent Application 2011-105663, Japanese Unexamined Patent Application 2011-168634, Japanese Unexamined Patent Application 2011-102354, and Japanese Unexamined Patent Application 2014-122316 described above, the silicone polycaprolactone copolymer particles of the present invention can be used in place of a part or all of these silicone-based particles, and there are cases where the feel during use as well as the production efficiency of the cosmetic material compositions proposed in these Patent Documents can be further improved. Note that it goes without saying that examples of cosmetic material compositions containing silicone particles (silicone rubber powder or the like) or silicone composite particles that can be blended with the silicone polycaprolactone copolymer particles of the present invention are not limited to the above, and formulations may be designed in which some or all of silicone particle components in commercially available cosmetic materials are replaced by the silicone elastomer particles of the present invention, using a technique common to a person of ordinary skill in the art.

Furthermore, the silicone polycaprolactone copolymer particles of the present invention can be applied to replace some or all of these silicone-based particles with respect to the applications and formulations of cosmetic material compositions disclosed in the aforementioned Patent Documents and the like, and the use therein is encompassed in the scope of the invention of the present application. As an example, the silicone polycaprolactone copolymer particles of the present invention may be and are preferably used in combinations of arbitrary components, such as cosmetic product media (aqueous media or oil-based media), oil-based media (including oil agents and volatile oil agents), water, colorants, pigments, ultraviolet light blocking components, alcohols, water-soluble polymers, film-forming agents, oil agents, oil-soluble gelling agents, organically modified clay minerals, surfactants, resins, salts, moisturizers, preservatives, antimicrobial agents, antioxidants, pH adjusters, chelating agents, refreshing agents, anti-inflammatory agents, skin brightening agents (such as whitening agents, cell activators, skin roughness improvement agents, blood circulation promoters, skin astringents, and anti-seborrheic agents), vitamins, amino acids, nucleic acids, hormones, inclusion compounds, bioactive substances, medicament active components, perfumes, and the like, by selecting a method or quantitative range similar to those disclosed in (International Patent Publication WO2017/191798).

In particular, the silicone polycaprolactone copolymer particles of the present invention have superior feel during use, texture, handling workability, storage stability, dispersibility, and high oil absorption properties that are equal or superior to conventionally known silicone particles, silicone composite particles coated with silsesquioxane, and silicone particles containing an oil agent. Therefore,
(1) cosmetic material compositions and formulations containing oil-based media such as oil agents and the like (oil-based cosmetic raw materials);
(2) cosmetic material compositions and formulations containing lipophilic ultraviolet light blocking components (such as octyl paramethoxycinnamate and the like); and
(3) cosmetic material compositions and formulations containing inorganic powders such as colorants, pigments, or the like,
can provide a particularly favorable appearance, feel of use, and the like. These specific formulations are further described in detail in the Examples.

In addition thereto, the silicone polycaprolactone copolymer particles of the present invention can be easily designed for aqueous dispersions. Therefore, in aqueous cosmetic material compositions and formulations, the silicone elastomer particles provide excellent formulation design freedom and blending stability, and thus can achieve a suitable feel during use. These specific formulations are further described in detail in the Examples.

The cosmetic material of the present invention can be easily manufactured by simply uniformly mixing the cosmetic product raw material of the present invention and other cosmetic product raw materials as described above. As mixing means, various mixing and kneading devices normally used in the manufacture of cosmetic products can be used. Examples of such devices include a homomixer, a paddle mixer, a Henschel mixer, a homodisper, a colloidal mixer, a propeller agitator, a homogenizer, an in-line continuous emulsifier, an ultrasonic emulsifier, and a vacuum kneading machine.

### Organic Resin Additives and Organic Resins, Paints, and Coating Agents

The silicone polycaprolactone copolymer particles of the present invention are also extremely useful as an organic resin additive due to the properties described above. Specifically, the silicone elastomer particles of the present invention have superior uniform dispersibility in organic resins and, if desired, excellent stress relief properties, and the like. In addition, the particles have very excellent handling workability and storage stability because aggregation is less likely to occur even after long-term storage. Furthermore, a member, paint film, or coating film obtained by curing an organic resin containing the silicone polycaprolactone copolymer particles has improved flexibility (including the softness of a coating layer), durability, and adhesion to and followability of the substrate, is particularly pliable, and has superior thermal shock resistance. Therefore, it is extremely useful as a highly functional organic resin, paint, or coating agent for use in electronic materials.

### Organic Resin

A curable organic resin composition or a thermoplastic resin is suitably exemplified as an organic resin containing the silicone polycaprolactone copolymer particles of the present invention. Of these, curable resins are suitable for electronic materials such as semiconductor substrates and the like. More specifically, examples of the curable organic resin composition include, phenolic resin, formaldehyde resin, xylene resin, xylene-formaldehyde resin, ketone-formaldehyde resin, furan resin, urea resin, imide resin, melamine resin, alkyd resin, unsaturated polyester resin, aniline resin, sulfone-amide resin, silicone resin, epoxy resin, and copolymer resins of these resins, and two or more of these curable resins can be combined. In particular, the curing resin is preferably at least one type selected from the group consisting of an epoxy resin, a phenolic resin, an imide resin, and a silicone resin. The epoxy resin can be any compound containing glycidyl or alicyclic epoxy groups, and examples include o-cresol novolac epoxy resins, phenol novolac epoxy resins, biphenyl epoxy resins, bisphenol A epoxy resins, bisphenol F epoxy resins, dicyclopentadiene epoxy resins, naphthalene epoxy resins, anthracene epoxy resins, naphthol aralkyl epoxy resins, polyvinylphenol epoxy resins, diphenylmethane epoxy resins, diphenylsulfone epoxy resins, triphenolalkane epoxy resins, cresol-naphthol co-condensation epoxy resins, bisphenylethylene epoxy resins, fluorene epoxy resins, stilbene epoxy resins, spiro-coumarone epoxy resins, norbornene epoxy resins, terpene epoxy resins, phenolcyclohexane epoxy resins, halogenated epoxy resins, imide-group-containing epoxy resins, maleimide-group-containing epoxy resins, allyl group-modified epoxy resins, and silicone-modified epoxy resins. Examples of this phenolic resin include a polyvinylphenol type, a phenol novolac type, a naphthol type, a terpene type, a phenol dicyclopentadiene type, a phenol aralkyl type, a naphthol aralkyl type, a triphenol alkane type, a dicyclopentadiene type, a cresol naphthol co-condensation type, and a xylene-naphthol co-condensation type. An example of a silicone resin is an epoxy-modified silicone resin generated by a reaction between an epoxy resin and a silanol group or a silicon-bonded alkoxy group in the silicone resin. Examples of the curing mechanisms of such curable resins are thermal curing, high energy beam curing such as ultraviolet light, radiation, and the like, moisture curing, condensation reaction curing, and addition reaction curing. The properties of such curable resins at 25 °C are not limited, and may be in either a liquid state or a solid state that softens upon heating.

Another optional component such as a curing agent, curing promoter, filler, photosensitizer, higher fatty acid metal salt, ester wax, plasticizer, or the like can be added to the organic resin containing the silicone polycaprolactone copolymer particles of the present invention. Examples of curing agents include: organic acids such as carboxylic acids, sulfonic acids, and the like and anhydrides thereof; organic hydroxy compounds; organic silicon compounds having a silanol group, an alkoxy group, or a halogeno group; and primary or secondary amino compounds, and two or more types can be combined. Examples of the curing promoter include: tertiary amine compounds, organic metal compounds such as aluminum, zirconium, and the like; organophosphorus compounds such as phosphine and the like; other heterocyclic amine compounds, boron complex compounds, organic ammonium salts, organic sulfonium salts, organic peroxides, and catalysts for hydrosilylation. Examples of these fillers include: fibrous fillers such as glass fiber, asbestos, alumina fiber, ceramic fiber composed of alumina and silica, boron fiber, zirconia fiber, silicon carbide fiber, metal fiber, polyester fiber, aramid fiber, nylon fiber, phenolic fiber, natural animal and plant fiber, and the like; and powdered fillers such as fused silica, precipitated silica, fumed silica, calcined silica, zinc oxide, calcined clay, carbon black, glass beads, alumina, talc, calcium carbonate, clay, aluminum hydroxide, barium sulfate, titanium dioxide, aluminum nitride, silicon carbide, magnesium oxide, beryllium oxide, Kaolin, mica, zirconia, and the like. Two or more of these can be combined. In the case of epoxy resins, it is particularly preferable to include an amine curing agent.

The silicone polycaprolactone copolymer particles of the present invention may be added as an additive to a thermoplastic resin other than those described above, and may be used as a modifier of physical properties such as surface lubricants, stress relief agents, and the like, or modifiers of optical properties such as light scattering agents and the like. The type of thermoplastic resin is not particularly limited, and may be at least one polymer selected from a group consisting of: polycaprolactone resins; polyester resins; polyether resins; polylactic acid resins; polyethylene, polypropylene, ethylenepropylene copolymers, and other polyolefin resins; polystyrene resins; styrene copolymers; tetrafluoroethylene and other fluorine-based polymers; polyvinyl ethers; and cellulose-based polymers, or a composite resin containing a combination of these. The silicone resin coated silicone elastomer particles of the present invention can be uniformly dispersed in these thermoplastic resins (including master batches) using a mixing device such as a biaxial or single-axis extruder, a kneader mixer, or the like, and may be molded into a desired shape, such as a film form or the like, for use.

The added amount of the silicone polycaprolactone copolymer particles of the present invention may be selected as appropriate according to the physical properties required of the organic resin, but is generally in a range of 0.1 to 30 parts by mass with respect to 100 parts by mass of the organic resin, and may be in a range of 0.5 to 10 parts by mass. The reason is that if the amount of the particles added is less than the lower limit, the performance such as stress relief properties for the resin or the like may become insufficient, and the pliability and thermal shock resistance of the resulting cured organic resin product may decrease, and in particular, the thermal shock resistance tends to decrease after moisture absorption. On the other hand, if the amount exceeds the upper limit, the organic resin or the paint/coating agent after blending may become thickened and the handling workability may decrease, and the mechanical properties of the resulting organic resin cured product tend to decrease.

Furthermore, the silicone polycaprolactone copolymer particles of the present invention are superior in stress relief when added to an organic resin, and thus may be added to an epoxy resin or the like for printed wiring boards to form a prepreg. Furthermore, a copper foil containing filler particles for a printed wiring board provided with a resin layer containing the silicone-polycaprolactone copolymer particles of the present invention on one side of the copper foil may be formed and used for a copper clad laminate (CCL) application.

### Paints, Coating Agents

Examples of paints and coating agents containing the silicone polycaprolactone copolymer particles of the present invention include ambient temperature curing types, ambient temperature drying types, and heat curing types, with examples based on the properties thereof including aqueous types, oil-based types, and powdered types, and examples based on the vehicle resin including polyurethane resin paint, butyral resin paint, long oil phthalate resin paint, alkyd resin paint, amino alkyd resin paint made up of amino resin and alkyd resin, epoxy resin paint, acrylic resin paint, phenol resin paint, silicone-modified epoxy resin paint, silicone-modified polyester resin paint, and silicone resin paint.

The added amount of the silicone polycaprolactone copolymer particles of the present invention can be selected as appropriate according to the physical properties required for the paint/coating agent, but in order to impart uniform and soft matting properties to the resulting coating film, the added amount is preferably in a range of 0.1 to 150 parts by mass with respect to 100 mass parts of solid content of the paint, more preferably in a range of 0.1 to 100 parts by mass, even more preferably 0.1 to 50 parts by mass, and particularly preferably 0.1 to 20 parts by mass. If the amount of the particles added is less than the lower limit, performance such as matting, adhesion, stress relief properties, and the like of the coating film may be insufficient. If the amount of the particles exceeds the upper limit, the organic resin and the paint/coating agent after blending may become thickened and the handling workability may decrease.

The paints and coating agents containing the silicone polycaprolactone copolymer particles of the present invention may contain: alcohols such as methanol, ethanol, and the like; ketones such as methyl ethyl ketone, methyl isobutyl ketone, and the like; esters such as ethyl acetate, butyl acetate, cellosolve acetate, and the like; amides such as N,N-dimethylformamide and the like; olefins such as hexane, heptane, octane, and the like; organic solvents such as toluene, xylene, and other aromatic hydrocarbons; known inorganic fillers such as reinforcing silica and the like; organic fillers; curing promoters; silane coupling agents; carbon black and other pigments; dyes; antioxidants; thickeners containing a macromolecular compound; flame retardants; and weather resistance imparting agents.

### As Eco-Friendly Material

As described above, unlike conventional non-biodegradable thermoplastic resin particles and silicone particle materials, the silicone polycaprolactone copolymer particles of the present invention are expected to be biodegradable in a biodegradable environment, where crosslinked structures formed between silicon atoms within the silicone polycaprolactone copolymer particles are at least partially cleaved, and the primary particles have a property of disintegrating with the generation of a polyorganosiloxane with a non-crosslinked structure. Therefore, use is possible as an "eco-friendly" cosmetic raw material with low environmental burden and environmental risk that complies with regulations on microplastics and the like, and appeal as a biodegradable and "eco-friendly" material is expected for users and consumers who are concerned about global environmental impact.

### EXAMPLES

The radical polymerizable polycaprolactone-modified silicone compound of the present invention, the silicone-polycaprolactone copolymer particles using the compound as a raw material, and the method for producing the compound will be described in detail with reference to examples and comparative examples. However, the present invention is not limited only to these examples. The viscosity in the examples is the value at 25°C. The properties of each particle were measured as follows. Note that in the examples and the like, unless otherwise specified, silicone particles is a generic term for particles made of a silicone cured product (cured silicone particles) (including the silicone-polycaprolactone copolymer particles of the present invention), and does not include emulsions.

### Average Primary Particle Diameter of Emulsion Particles

The emulsion before the addition of the radical polymerization initiator was measured using a laser diffraction particle diameter distribution analyzer (LS-230 from Beckman Coulter), and the median diameter (particle diameter corresponding to 50% of the cumulative distribution, 50% particle diameter) was used as the average particle diameter.

### Average Secondary Particle Diameter of Elastomer Particles (Powder)

Using ethanol as a dispersion medium, the particle diameter of the cured silicone particles was measured with a laser diffraction particle diameter distribution analyzer (Mastersizer 3000 from Malvern Panalytical), and the median diameter of the cured elastomer particles in ethanol (particle diameter corresponding to 50% of the cumulative distribution, D90, µm) and the arithmetic dispersion (particle diameter distribution SD, µm²) values were obtained. For the measurement sample, cured silicone particles (1 g) and ethanol (100 mL) were dispersed in a 300 mL cup using stirring blades and an ultrasonic vibrator.

### Synthesis Example 1: SiH double terminated side chain methacrylate-modified siloxane

A four-necked separable flask was charged with 89.83 parts by weight of a SiH double terminated siloxane having a degree of polymerization of 43, 10.17 parts by weight of 3-methacryloxypropylmethyldimethoxysilane, and 0.01 parts by weight of MEHQ (hydroquinone monomethyl ether, a polymerization inhibitor). The mixture was heated and stirred while blowing nitrogen gas, and when the temperature reached 50°C, 0.05 parts by weight of trifluoromethanesulfonic acid and 2.37 parts by weight of water were added. After reacting at 65°C for 1 hour, the liquid temperature was heated to 70°C. Furthermore, the pressure was reduced to 100 mmHg to remove the methanol produced as a by-product for about 1 hour. Thereafter, the mixture was reacted at 70°C for 3 hours. After the reaction, ammonia gas was bubbled to neutralize the trifluoromethanesulfonic acid, and the generated salt was removed by filtration. The filtrate was subjected to reduced pressure processing at 150°C for 3 hours to remove volatile components. C, Si-NMR analysis revealed that a SiH double terminated side chain methacryl-modified silicone polymer had a dimethylsiloxane unit content of 45.3, a methacryl group-introduced siloxane unit content of 2.0, and a viscosity of 55.4 cSt.

### Synthesis Example 2: Synthesis of vinyl-terminated polycaprolactone

A four-necked separable flask was charged with 9.50 parts by weight of ethylene glycol monoallyl ether, 75.20 parts by weight of ε-caprolactone, 15.3 parts by weight of chloroform, and 1157 ppm of triazabicyclodecene. The mixture was stirred for 12 hours while bubbling nitrogen gas, and 5000 ppm of benzoic acid was added. Next, the mixture was washed with a saturated aqueous solution of sodium hydrogen carbonate. The chloroform phase was collected and the chloroform was removed under reduced pressure. A white waxy polymer was obtained. H-NMR analysis revealed that the following double terminated alcohol-modified and side chain methacryl-modified siloxane was obtained.

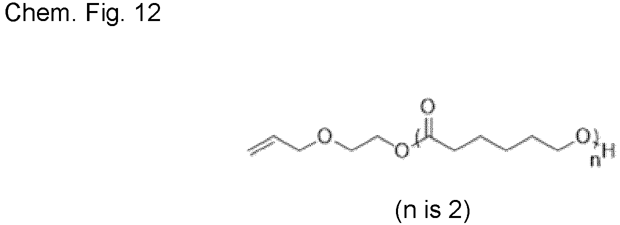

### Example 1: Double terminated methacryl and polycaprolactone-modified compound

A four-necked separable flask was charged with 35.50 parts by weight of a SiH-terminated siloxane having a degree of polymerization of 43, 31.90 parts by weight of the vinyl-terminated polycaprolactone, 32.60 parts by weight of toluene, and 0.01 part by weight of a 2% sodium acetate methanol solution. The mixture was heated to 60°C while bubbling nitrogen gas. An isopropyl alcohol solution of chloroplatinic acid (in an amount such that the platinum metal in the composition was 10 ppm by mass) was added, and the mixture was allowed to react for 5 hours. The toluene was removed under reduced pressure to provide a white waxy polymer.

37.00 parts by weight of the obtained polymer, 53.40 parts by weight of chloroform, 6.20 parts by weight of potassium carbonate, and 63 ppm of hydroquinone monomethyl ether were added. The mixture was stirred while bubbling nitrogen gas, and then 3.40 parts by weight of acryloyl chloride was added dropwise. The reaction was carried out for 5 hours while controlling the heat generation so that the temperature did not exceed 30°C. The chloroform soluble fraction was collected, and the chloroform was removed under reduced pressure. The resulting polymer was found by H-NMR analysis to have the following structure. 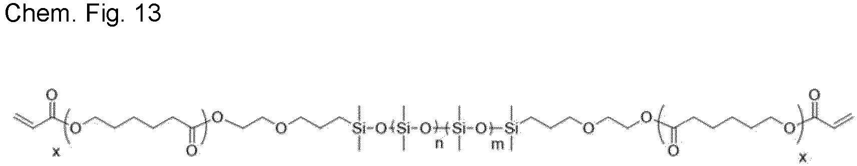 (In the formula, a methyl group is bonded to the line (-) on the silicon atom, and (m + n), and x are 43.0 and 2, respectively.)

### Example 1: Side chain methacryl-modified siloxane and double terminated polycaprolactone-modified compound

A four-necked separable flask was charged with 40.60 parts by weight of Synthesis Example 1, 25.70 parts by weight of the vinyl-terminated polycaprolactone, 33.70 parts by weight of toluene, and 0.01 part by weight of a 2% sodium acetate methanol solution. The mixture was heated to 60°C while bubbling nitrogen gas. An isopropyl alcohol solution of chloroplatinic acid (in an amount such that the platinum metal in the composition was 10 ppm by mass) was added, and the mixture was allowed to react for 5 hours. The toluene was removed under reduced pressure to provide a white waxy polymer. The resulting polymer was found by H-NMR analysis to have the following structure. 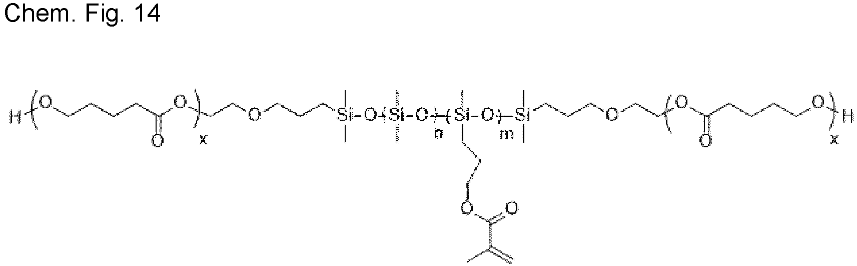 (In the formula, a methyl group is bonded to the line (-) on the silicon atom, and m, n, and x are 2.0, 45.3, and 2, respectively.)

### Comparative Example 1: Acrylic-modified polycaprolactone compound

A four-neck separable flask was filled with 18.81 parts by weight of Placcel 205 (trade name, manufactured by Daicel Corporation, diol type polycaprolactone, mol. wt.: 530), 18.81 parts by weight of chloroform, 8.77 parts by weight of triethyl amine and 0.03 parts by weight of hydroquinone monomethyl ether. The mixture was stirred while bubbling nitrogen gas. 6.55 parts by weight of acryloyl chloride was added dropwise. The temperature was adjusted to stay under 30°C. After dropwise addition was completed, the mixture was reacted for 1 hour. Furthermore, the liquid temperature was raised to 50°C, and the mixture was aged for about 2 hours. Next, 28.21 parts by weight of water was added and stirred well. Thereafter, the mixture was transferred to a separatory funnel, and the bottom phase containing the modified polycaprolactone was removed. Furthermore, 18.81 parts by weight of water was added to make the mixture uniform. The sample was again transferred to a separatory funnel and left overnight for further separation. After sitting overnight, the solution was removed and transferred to another four-necked flask, and the chloroform was removed under reduced pressure to obtain a clear orange polymer. Based on H-NMR analysis, a peak derived from an acrylic group appeared in the polymer, and it was confirmed that the polymer was polycaprolactone having a terminal modified with an acrylic group ((meth)acryl-modified polycaprolactone compound) having the following structure. 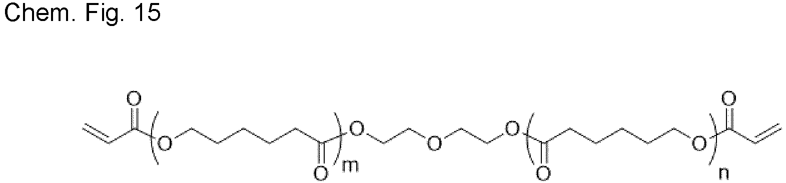 (In the formula, m + n = 3.7)

In the above Example 1 and Example 2, the radical polymerizable polycaprolactone-modified silicone compound according to the present invention was obtained. Hereinafter, Example 3 and Example 4 will be used to show examples of the production of silicone-polycaprolactone copolymer particles obtained using the above-mentioned side chain (meth)acrylic-modified siloxane and double terminated polycaprolactone-modified compound and the above-mentioned double terminated (meth)acrylic and polycaprolactone-modified siloxane compound as raw materials. Note that Comparative Example 1 is non-silicone-based polymer particle obtained using only a (meth)acrylic-modified polycaprolactone compound as a raw material.

### Example 3: Silicone and polycaprolactone copolymer particle No. 1

The double terminated (meth)acryl and polycaprolactone-modified siloxane compound of Example 1 was dispersed in an aqueous solution at 25°C containing 0.23 parts by mass of GOHSENOL EG-05C (manufactured by Mitsubishi Chemical: polyvinyl alcohol), 0.47 parts by mass of GOHSENOL EG-18P (manufactured by Mitsubishi Chemical: polyvinyl alcohol), and 46 parts by mass of pure water, the resulting mixture was uniformly emulsified using a colloid mill, and then 526 parts by mass of pure water was added to dilute the mixture to prepare an emulsion. After heating in a 1 L flask to 70°C, an aqueous solution of 0.5 g potassium persulfate (manufactured by Sigma-Aldrich) dissolved in 9.5 g water was added dropwise over one minute. The emulsion was subjected to radical polymerization at 70°C for 3 hours, the temperature was further increased to continue the reaction at 80°C for 2 hours, and then 0.8 g of aminomethylpropanediol was then added to end the reaction, thereby preparing a uniform aqueous suspension of copolymer particles. The aqueous suspension was then filtered and washed with 200 mL of ethanol and 100 mL of acetone. The residue was dried in an oven at 70°C for 5 hours to obtain silicone and polycaprolactone copolymer particles No. 1. The average primary particle size and average secondary particle size of the obtained particles were 4.84 µm and 273 µm, respectively.

### Example 4: Silicone and polycaprolactone copolymer particles No. 2

The same procedure as in Example 3 was performed, except that the material to be emulsified was changed to the side-chain methacryl-modified siloxane and double terminated polycaprolactone-modified compound of Example 2, to obtain the silicone and polycaprolactone copolymer particles No. 2. The average primary particle size and average secondary particle size of the obtained particles were 3.48 µm and 141 µm, respectively.

### Comparative Example 2 (Radical Polymerization Polymer Particles)

Non-silicone polymer particles were obtained in the same manner as in Example 3, except that a polyorganosiloxane component was not used and 100 mass parts of only (meth)acryl-modified polycaprolactone compound No. 1 was used. The average primary particle size and the average secondary particle size were 2.91 µm and 70.0 µm, respectively.

The average primary and secondary particle diameters of each particle obtained from the above Examples 3 and 4 and Comparative Example 2 are summarized in Table 1 below.

**Table 1**

| | Average primary particle diameter (µ m) | Average secondary particle diameter (µ m) |
|---|---|---|
| Silicone-polycaprolactone copolymer particles No. 1 | 4.84 | 273 |
| Silicone-polycaprolactone copolymer particles No. 2 | 3.48 | 141 |
| Radical polymerization type polymer particles of comparative example 2 | 2.91 | 70.0 |

### Cosmetic Material Formulation Example

The following are formulation examples of cosmetic materials of the present invention that can contain silicone elastomer particles, which is one aspect of the present invention. However, the present invention is not limited thereto.

Examples 5 and 6 and Comparative Examples 3 and 4

The feel of use of water-in-oil sunscreens having the compositions shown in the following table was compared and evaluated by a panel of experts. In addition, the SPF and PA values were measured and compared using an SPF analyzer.

### Evaluation of Tactile Sensation

The 18 panelists evaluated the spreadability and whitening properties when the samples were applied to the inner forearm.

**Table 2**

| Evaluation results | Evaluation Indicators |
|---|---|
| ○ | 12 or more of 18 respondents said spreadability is favorable and white residue is less likely to remain |
| Δ | 7 to 11 out of 18 respondents said spreadability is favorable and white residue is less likely to remain |
| X | 6 or less of 18 respondents said spreadability is favorable and white residue is less likely to remain |

**Table 3**

| Phase | Component | Product name and supplier | COMPARATIVE EXAMPLE 3 | COMPARATIV E EXAMPLE 4 | EXAMPLE 5 | EXAMPLE 6 |
|---|---|---|---|---|---|---|
| A | Zinc oxide dispersion (50% zinc oxide diluted with 2cs dimethicone) | | 20 | 20 | 20 | 20 |
| | Titanium dioxide dispersion (50% titanium dioxide diluted with 2cs dimethicone) | | 36 | 36 | 36 | 36 |
| | Dimethicone | XIAMETE R^{™} PMX-200 2cs | 9.8 | 6.8 | 6.8 | 6.8 |
| | Caprylyl methicone | DOWSIL^{™} FZ-3196 | 10 | 10 | 10 | 10 |
| | Silicone emulsifiers | DOWSIL^{™} ES-5300 Formulatio n Aid | 2 | 2 | 2 | 2 |
| | Dimethicone crosspolymer | DOWSIL^{™} EP-9610 Cosmetic Powder | | 3 | | |
| | Silicone-polycaprolactone copolymer particles No. 1 | | | | 3 | |
| | Silicone-polycaprolactone copolymer particles No. 2 | | | | | 3 |
| B | Sodium citrate | | 0.2 | 0.2 | 0.2 | 0.2 |
| | Sodium chloride | | 0.5 | 0.5 | 0.5 | 0.5 |
| | BG (Butylene glycol) | | 3 | 3 | 3 | 3 |
| | Water | | 17.8 | 17.8 | 17.8 | 17.8 |
| C | Preservative | Euxyl PE9010 available from Schülke & Mayr | 0.7 | 0.7 | 0.7 | 0.7 |
| Evaluation of tactile sensation | | | × | × | ○ | ○ |
| SPF value | | | 39 | 22 | 47 | 102 |
| PA value | | | +++ | +++ | +++ | ++++ |

### Method of preparation

1. Phase A is mixed.
2. Phase B is mixed.
3. Phases A and B are stirred until uniform.

### SPF value and PA value

An evaluation target (sunscreen cosmetic composition) was uniformly applied to HELIOPLATE HD6 (available from HelioScreenLab) so as to be 2 mg/cm² to measure the SPF value and PA value using the SPF measuring device UV-1000S (available from Labsphere). The values listed in Table 3 are average values excluding the maximum value and the minimum value by performing 10 measurements on each of three test samples.

As shown in Table 3, the water-in-oil sunscreen containing silicone-polycaprolactone copolymer particle No. 1 of the present invention (Example 3) and the silicone polycaprolactone copolymer particles No. 2 (Example 4) were evaluated to have a good feel and SPF improving effect, unlike the water-in-oil sunscreen not containing these particles (Comparative Example 3) or containing other particles (Comparative Example 4).

### Examples 7 and 8 and Comparative Examples 5 and 6

The panelists compared and evaluated the feel during use of oil-in-water skin cream in which the silicone elastomer particles in the compositions listed in Table 5 were used.

### Evaluation of Tactile Sensation

Eighteen panelists applied the samples to the inside of their forearms and evaluated the spreadability and smoothness according to the criteria in Table 4 below.

**Table 4**

| Evaluation results | Evaluation Indicators |
|---|---|
| ○ | 12 or more of 18 persons responded that spreadability was favorable and there was a moist feel after drying |
| Δ | 7 to 11 of 18 persons responded that spreadability was favorable and there was a moist feel after drying |
| × | 6 or fewer of 18 persons responded that spreadability was favorable and there was a moist feel after drying |

**Table 5**

| Phase | Component | Product name and supplier | COMPARATIVE EXAMPLE 5 | EXAMPLE 7 | EXAMPLE 8 | COMPARATIVE EXAMPLE 6 |
|---|---|---|---|---|---|---|
| A | Silicone emulsifier premix | ACULY N^{™} Siltouch Rheolog y Modifier | 2 | 2 | 2 | 2 |
| | Glyceryl tri(capric acid/caprylic acid) | | 2 | 2 | 2 | 2 |
| | Almond oil | | 6 | 6 | 6 | 6 |
| | Silicone-polycaprolactone copolymer particles No. 1 | | | 1 | | |
| | Silicone-polycaprolactone copolymer particles No. 2 | | | | 1 | |
| | Radical polymerization type polymer particles of comparative example 2 | | | | | 1 |
| B | BG (Butylene glycol) | | 5 | 5 | 5 | 5 |
| | Water | | Residual | Residual | Residual | Residual |
| C | Preservative | Euxyl PE9010 available from Schülke & Mayr | 0.7 | 0.7 | 0.7 | 0.7 |
| Evaluation of tactile sensation | | | × | ○ | ○ | Δ |

### Method of preparation

1. Phase A is mixed.
2. Phase B is mixed.
3. Phase B is slowly added while stirring Phase A.
4. Phase C is added to 3 above and then stirred until uniform.

As shown in Table 5, the water-in-oil sunscreens using silicone and polycaprolactone copolymer particles No. 1 (Example 3) and silicone and polycaprolactone copolymer particles No. 2 (Example 4) of the present invention were evaluated as having good spreadability and a moist feel after drying, unlike skin creams that did not contain elastomer particles (Comparative Example 6) and those that used other particles (Comparative Example 5).

## Claims

1. A radical polymerizable polycaprolactone-modified silicone compound, comprising:
two or more modified polycaprolactone structures bonded to a silicon atom either directly or via a divalent linking group, the structures expressed by the following structural formula (1): 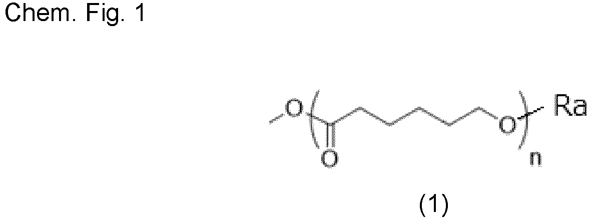
{where n is a number ranging from 1 to 5;
Ra is a group selected from hydrogen atoms and (meth)acrylic terminal groups expressed by -R₁-CR²=CH₂ (where R¹ is a carbonyl group or a divalent linking group containing one carbonyl group, and R² is a hydrogen atom or a methyl group)};
and if the number of (meth)acrylic terminal groups contained in the modified polycaprolactone structure in the molecule is less than two, the molecule further includes two or more (meth)acrylic terminal groups expressed by -R³-CR⁴=CH₂ and bonded to silicon atoms (where R³ is a carbonyl group or a divalent linking group containing one carbonyl group, and R⁴ is a hydrogen atom or a methyl group).

2. The radical polymerizable polycaprolactone-modified silicone compound according to claim 1, having a straight chain or branched chain polysiloxane structure, wherein the molecular chain terminal or molecular chain side chain includes:
two or more modified polycaprolactone structures bonded to a silicon atom either directly or via a bivalent linking group, the structures expressed by the following structural formula (1): 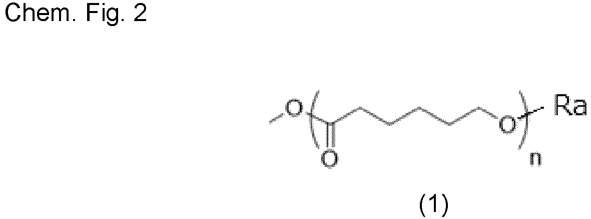
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R² is a hydrogen atom or a methyl group)};
and if the number of (meth)acrylic terminal groups contained in the modified polycaprolactone structure in the molecule is less than two, the molecule includes two or more (meth)acrylic terminal groups expressed by - R³-CR⁴=CH₂ and bonded to silicon atoms (where R³ is a carbonyl group or a divalent linking group containing one carbonyl group, and R⁴ is a hydrogen atom or a methyl group).

3. The radical polymerizable polycaprolactone-modified silicone compound according to claim 1 or claim 2, expressed by the following structural formula (2): 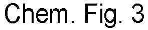 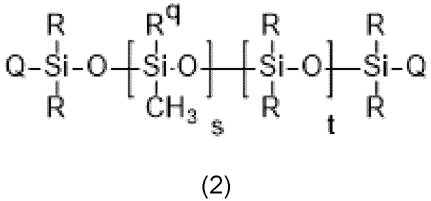
{where Q is a modified polycaprolactone structure expressed by the following structural formula (1), bonded to a silicon atom either directly or through a divalent linking group: 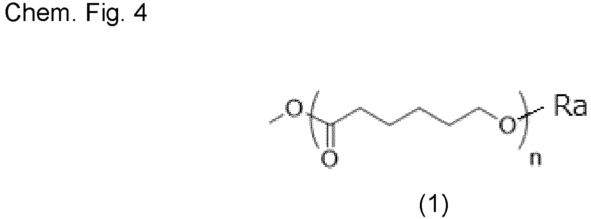
{where n is a number ranging from 1 to 5;
Ra is a group selected from hydrogen atoms and (meth)acrylic terminal groups expressed by -R¹-CR²=CH₂ (where R¹ is a carbonyl group or a divalent linking group containing one carbonyl group, and R₂ is a hydrogen atom or a methyl group)};
R^{q} is a (meth)acrylic terminal group bonded to a silicon atom and expressed by -R³-CR⁴=CH₂ (where R³ is a carbonyl group or a divalent linking group containing one carbonyl group, and R⁴ is a hydrogen atom or a methyl group);
R is an alkyl group or an aryl group having 1 to 20 carbon atoms;
s is a number ranging from 0 to 50, and t is a number ranging from 1 to 500;
however, when Ra is a hydrogen atom in one or both of the terminal groups Q, s is a positive number and indicates having at least two (meth)acrylic terminal groups in the molecule}.

4. The radical polymerizable polycaprolactone-modified silicone compound according to claim 3, wherein the following condition (I) or (II) is satisfied in the structural formula (2).
(I) In both terminal groups Q, Ra is a hydrogen atom, s is a number in a range of 2 to 50, and t is a number in a range of 1 to 500;
(II) In both of the terminal groups Q, Ra is a (meth)acrylic terminal group expressed by -R¹-CR²=CH₂ (where R¹ is a carbonyl group or a divalent linking group containing one carbonyl group, and R² is a hydrogen atom or a methyl group), s is a number in a range of 0 to 50, and t is a number in a range of 1 to 500.

5. The radical polymerizable polycaprolactone-modified silicone compound according to any one of claims 1 to 4, which is a raw material for synthesizing silicone-polycaprolactone copolymer particles.

6. A method for producing the radical polymerizable polycaprolactone-modified silicone compound according to any one of claims 1 to 4, comprising a step of:
performing a hydrosilylation reaction between
(a) an organohydrogenpolysiloxane having at least 2 silicon-bonded hydrogen atoms in each molecule; and
(b) a polycaprolactone compound having a vinyl terminal structure expressed by the following structural formula (1') 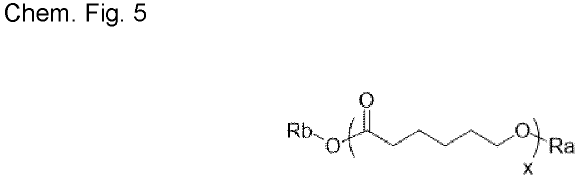 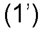
{wherein x is a number ranging from 1 to 5,
Ra is a group selected from hydrogen atoms and (meth)acrylic terminal groups expressed by -R₁-CR²=CH₂ (where R¹ is a carbonyl group or a divalent linking group containing one carbonyl group, and R² is a hydrogen atom or a methyl group),
Rb is CH₂=CH(Rc)-R'- (Rc is a hydrogen atom or a methyl group, and R' is a divalent organic group having 1 to 20 carbon atoms which may contain an oxygen atom)}
in the presence of
(c) a hydrosilylation reaction catalyst.

7. The method for producing the radical polymerizable polycaprolactone-modified silicone compound according to claim 6, wherein in the structural formula (1'), Ra is a hydrogen atom, and the polycaprolactone-modified silicone compound obtained after the hydrosilylation reaction is further subjected to
(d) a step of reacting a (meth)acryloyl chloride compound expressed by Cl-C(=O)-R¹ -CR² =CH₂ (wherein R¹ is a chemical bond between CH and C(=O) or a divalent organic group having 1 to 20 carbon atoms, and R² is a hydrogen atom or a methyl group) in the presence of a basic catalyst.

8. The method for producing radical polymerizable polycaprolactone-modified silicone compound according to claim 6, where the aforementioned component (a) is (a') an organohydrogenpolysiloxane having in the molecule at least two silicon-bonded hydrogen atoms and a (meth)acrylic terminal group bonded to a silicon atom and expressed by -R³-CR⁴=CH₂ (where R³ is a carbonyl group or a divalent linking group containing one carbonyl group, and R⁴ is a hydrogen atom or a methyl group).

9. Silicone-polycaprolactone copolymer particles having a structure in which at least two silicon atoms in the particle are crosslinked by a radical polymerization reaction of the radical polymerizable polycaprolactone-modified silicone compound according to any one of claims 1 to 4.

10. The silicone-polycaprolactone copolymer particles according to claim 9, obtained by radical polymerization of (A) 100 parts by mass of the radical polymerizable polycaprolactone-modified silicone compound according to any one of claims 1 to 4, in the presence of
(B) 0.1 to 10 parts by mass of a radical polymerization initiator.

11. The silicone-polycaprolactone copolymer particles according to claim 9 or claim 10, obtained by crosslink reacting in water crosslinkable emulsified particles made by emulsifying in water a crosslinkable silicone composition that can be crosslinked by a radical polymerization reaction, the composition containing (A) 100 parts by mass of the radical polymerizable polycaprolactone-modified silicone compound according to any one of claims 1 to 4; and (B) at least 0.1 to 10 parts by mass of a radical polymerization initiator.

12. The silicone polycaprolactone copolymer particles according to any one of claims 9 to 11, wherein the average primary particle diameter as measured by a laser diffraction scattering method is 0.5 to 20 µm.

13. The silicone-polycaprolactone copolymer particles according to any one of claims 9 to 11, wherein the radical polymerizable polycaprolactone-modified silicone compound used for particle formation has a JIS-A hardness of the compound, measured after curing into a sheet, in a range of 10 to 80.

14. The silicone polycaprolactone copolymer particles according to any one of claims 9 to 11, further comprising a structure in which some or all of the surface thereof is covered with one or more types selected from organopolysiloxane resins, silica, and other silicone elastomer particles.

15. Silicone-polycaprolactone copolymer particles according to any one of claims 9 to 11, wherein the particles have a mesoporous structure.

16. Silicone-polycaprolactone copolymer particles according to any one of claims 9 to 11, wherein the particles contain an oil agent that is liquid at 40°C.

17. Silicone-polycaprolactone copolymer particles according to any one of claims 9 to 16, that provide biodegradability.

18. The silicone-polycaprolactone copolymer particles according to any one of claims 9 to 16, wherein a divalent organic group having a partial structure formed by a radical polymerization reaction of the (meth)acrylic-modified moiety in the particle is active in a biodegradable reaction, and the crosslinked structure formed between silicon atoms in the particle is at least partially cleaved in a biodegradable environment, and the primary particles are disintegrated in conjunction with generation of polyorganosiloxane having a non-crosslinked structure.

19. A cosmetic material raw material, comprising the silicone-polycaprolactone copolymer particles according to any one of claims 9 to 18.

20. A cosmetic material composition, comprising the silicone-polycaprolactone copolymer particles according to any one of claims 9 to 18.

21. An organic resin additive, comprising the silicone-polycaprolactone copolymer particles according to any one of claims 9 to 18.

22. An organic resin, comprising the silicone-polycaprolactone copolymer particles according to any one of claims 9 to 18.

23. A method for producing the silicone-polycaprolactone copolymer particles according to any one of claims 9 to 18, comprising the following steps (I) and (II):
Step (I): a step of forming crosslinkable emulsified particles, by emulsifying in water
(A) the radical polymerizable polycaprolactone-modified silicone compound according to any one of claims 1 to 4, in the presence of
(B) a radical polymerization initiator;
Step (II): a step of curing the crosslinkable emulsified particles obtained in step (I) in the presence of (B) a radical initiator to obtain silicone-polycaprolactone copolymer particles.
